# EUROPÄISCHE PATENTSCHRIFT

(11) **EP 0 711 268 B1**
(45) Veröffentlichungstag und Bekanntmachung des Hinweises auf die Patenterteilung: **28.10.1998**
(21) Anmeldenummer: 94924233.3
(22) Anmeldetag: 05.07.1994
(51) Int. Cl.: C07C 43/11, C07C 41/03, C01B 13/14, C01F 7/00

(54) **VERFAHREN ZUR HERSTELLUNG VON ALKOXYLIERUNGSPRODUKTEN IN GEGENWART VON MIT ADDITIVEN MODIFIZIERTEN MISCHHYDROXIDEN**
METHOD FOR PREPARING ALKOXYLATION PRODUCTS IN THE PRESENCE OF ADDITIVE-MODIFIED MIXED HYDROXIDES
PROCEDE DE PREPARATION DE PRODUITS D'ALCOXYLATION EN PRESENCE D'HYDROXYDES MIXTES MODIFIES PAR DES ADDITIFS

(30) Priorität: 28.07.1993 DE 4325237
(43) Veröffentlichungstag der Anmeldung: 15.05.1996
(73) Patentinhaber: BASF AKTIENGESELLSCHAFT, 67056 Ludwigshafen (DE)
(72) Erfinder: WOLF, Gerhard, D-68775 Ketsch (DE); BURKHART, Bernd, D-67112 Mutterstadt (DE); LAUTH, Guenter, D-67229 Grosskarlbach (DE); TRAPP, Horst, D-68723 Plankstadt (DE); OFTRING, Alfred, D-67098 Bad Duerkheim (DE)
(86) Internationale Anmeldenummer: EP9402195
(87) Internationale Veröffentlichungsnummer: WO9504024

(56) Entgegenhaltungen:
- EP-A- 0 063 631
- WO-A-92/11224
- WO-A-92/20619
- DE-A- 4 010 606
- DE-A- 4 034 305
- DE-A- 4 140 746
- US-A- 4 774 212
- Römpps Chemie-Lexicon,achte,neubearbeite und erweitere Auflage,Seite122

## Beschreibung

Die vorliegende Erfindung betrifft ein verbessertes Verfahren zur Herstellung von Alkoxylierungsprodukten durch Umsetzung von Verbindungen mit aktiven Wasserstoffatomen mit C₂- bis C₄-Alkylen-oxiden in Gegenwart eines mit bestimmten Additiven modifizierten, aus Polykationen aufgebauten Mischhydroxids als Alkoxylierungskatalysator. Da ein Teil dieser Mischhydroxide neue Verbindungen darstellen, betrifft die Erfindung weiterhin diese neuen Mischhydroxide sowie Verfahren zu ihrer Herstellung.

Alkoxylierungsprodukte mit einer engen Molgewichtsverteilung oder Homologenverteilung, sogenannte "Narrow-cut"-, "Narrow-range"- oder "Peaked"-Alkoxylate, gewinnen in zunehmendem Maße an Bedeutung, da sie gegenüber normalen Alkoxylaten mit breiter Verteilung, die üblicherweise mit Alkalimetallhydroxiden oder Bortrifluorid-Addukten als Katalysatoren hergestellt werden, verbesserte anwendungstechnische Eigenschaften aufweisen. Sie finden hauptsächlich Verwendung als Tenside in Wasch-, Reinigungs- und Körperpflegemitteln, daneben aber auch in der Papier- und der Textilfaserindustrie. Effiziente Synthesewege für derartige "Narrow-cut"-Alkoxylate werden daher vordringlich gesucht.

Die DE-A 40 10 606 (1) betrifft die Verwendung von mit Anionen einer aliphatischen C₄- bis C₄₄-Dicarbonsäure oder einer aliphatischen C₂- bis C₃₄-Monocarbonsäure hydrophobierten Hydrotalkiten als Ethoxylierungs- oder Propoxylierungskatalysatoren von Verbindungen mit aktiven Wasserstoffatomen oder von Fettsäureestern. Die mit diesen Katalysatoren erhaltenen Alkoxylierungsprodukte weisen eine enge Homologenverteilung auf.

Aus der DE-A 40 34 305 (2) sind mit Anionen einer aliphatischen C₄- bis C₄₄-Dicarbonsäure oder einer aliphatischen C₂- bis C₃₄-Mono-carbonsäure hydrophobierte Doppelschichthydroxid-Verbindungen mit Magnesium, Zink, Calcium, Eisen, Kobalt, Kupfer, Cadmium, Nickel oder Mangan als zweiwertigem Metall und Aluminium, Eisen, Chrom, Mangan, Wismut oder Cer als dreiwertigem Metall und mit Carbonat, Hydrogencarbonat, Sulfat, Nitrat, Nitrit, Phosphat, Hydroxid oder Halogenid als anorganischem Anion neben Hydroxid bekannt. Diese Verbindungen werden als Alkoxylierungskatalysatoren für Verbindungen mit aktiven Wasserstoffatomen oder Fettsäureester empfohlen.

Die WO-A 92/11224 (3) betrifft ein Alkoxylierungsverfahren von Alkoholen zu Glykolethern unter Verwendung eines anionischen Doppelhydroxid-Tones wie Hydrotalkit mit im wesentlichen intakter Schichtstruktur als Alkoxylierungskatalysator, welcher zwischen den Schichten Anionen des reagierenden Alkohols eingebaut enthält. Als Alkohole werden hierfür aliphatische, cycloaliphatische oder aromatische Alkohole, vorzugsweise mit bis zu 8 C-Atomen, z.B. Methanol, Ethanol, Cyclohexanol oder Phenol, genannt.

Die WO-A 92/20619 offenbart mit Polymeren modifizierten Hydrotalkit. Als zusätzliche Additive werden Polyole genannt. Der beschriebene Hydrotalkit wird zur Stabilisierung von halogenhaltigen Kunststoffen eingesetzt.

In der DE-A 41 40 746 wird ein Verfahren zur Sorption von wenig polaren und unpolaren organischen Substanzen aus polarem Medium beschrieben, bei dem ein anionisch durch Fettsäuren hydrophobierter Hydrotalkit eingesetzt wird.

Aus der EP-A 063 631 ist eine Detergenz-Dispersant-Zusammensetzung für Schmieröle oder Brennstoffe bekannt, welche einen mit einem anionischen oberflächenaktiven Mittel modifizierten Hydrotalkit enthält. Als anionische oberflächenaktive Mittel werden Alkalimetallsalze von höheren Fettsäuren und von aromatischen Sulfonsäuren genannt.

Die genannten Alkoxylierungskatalysatoren weisen aber noch eine Reihe von Mängeln auf. Insbesondere der Gehalt an als Nebenprodukt bei der Alkoxylierung entstehendem Polyalkylenglykol sowie an nicht alkoxylierter Ausgangsverbindung sind noch zu hoch. Die Molekulargewichtsverteilung ist ebenfalls noch zu breit.

Aufgabe der vorliegenden Erfindung war es daher, effizientere und gleichzeitig dabei einfach und wirtschaftlich herzustellende Alkoxylierungskatalysatoren bereitzustellen, die die oben geschilderten Mängel nicht mehr aufweisen.

Demgemäß wurde ein Verfahren zur Herstellung von Alkoxylierungsprodukten durch Umsetzung von Verbindungen mit aktiven Wasserstoffatomen mit C₂- bis C₄-Alkylenoxiden in Gegenwart eines mit Additiven modifizierten, aus Polykationen aufgebauten Mischhydroxids der allgemeinen Formel I oder II

[M(II)₁₋ₓM(III)ₓ(OH)₂]A_{x/n} · m L (I)

[LiAl₂(OH)₆]A_{1/n} · m L (II)

in denen
- M(II): mindestens ein zweiwertiges Metallion,
- M(III): mindestens ein dreiwertiges Metallion,
- A: mindestens ein anorganisches Anion und
- L: ein organisches Lösungsmittel oder Wasser bedeutet,
- n: die Wertigkeit des anorganischen Anions A oder bei mehreren Anionen A deren mittlere Wertigkeit bezeichnet und
- x: einen Wert von 0,1 bis 0,5 und
- m: einen Wert von 0 bis 10 annehmen kann,
als Alkoxylierungskatalysator gefunden, welches dadurch gekennzeichnet ist, daß das Mischhydroxid als Additive
(a) aromatische oder heteroaromatische Mono- oder Polycarbonsäuren oder deren Salze,
(b) aliphatische Mono- oder Polycarbonsäuren oder deren Salze aus der Gruppe Phenyl-C₂- bis C₄-alkansäuren, Cyclopentancarbonsäure, Cyclohexancarbonsäure, Cyclohexyl-C₂- bis C₄-alkansäuren, Zimtsäure, o- oder p-Chlorzimtsäure und Pyridylessigsäuren,
(c) Halbester von Dicarbonsäuren oder deren Salze,
(d) Carbonsäureanhydride,
(e) aliphatische oder aromatische Sulfonsäuren oder deren Salze,
(f) C₈- bis C₁₈-Alkylsulfate,
(g) C₈- bis C₅₀-n-Alkane,
(h) Polyetherole oder Polyetherpolyole oder
(j) Alkohole oder Phenole, welche nicht zwischen den Schichten des Mischhydroxids I oder II eingebaut sind,
enthält.

Als zweiwertige Metallionen M(II) kommen insbesondere Zink, Calcium, Strontium, Barium, Eisen, Kobalt, Nickel, Cadmium, Mangan, Kupfer sowie vor allem Magnesium in Betracht.

Als dreiwertige Metallionen M(III) kommen insbesondere Eisen, Chrom, Mangan, Wismut, Cer sowie vor allem Aluminium in Betracht.

Als anorganische Anionen A eignen sich insbesondere Hydrogencarbonat, Sulfat, Nitrat, Nitrit, Phosphat, Chlorid, Bromid, Fluorid, Hydroxid sowie vor allem Carbonat.

Die Wertigkeit oder die mittlere Wertigkeit n des oder der anorganischen Anionen A liegt normalerweise im Bereich von 1 bis 3.

L bedeutet ein organisches Lösungsmittel, insbesondere einen Alkohol wie Methanol, Ethanol oder Isopropanol, oder vor allem Wasser.

Es ist eine große Anzahl aus Polykationen aufgebauter Mischhydroxide zwischen zwei- und dreiwertigen Metallen bekannt. Daneben sind auch derartige Mischhydroxide zwischen ein- und dreiwertigen Metallen bekannt ("Lithiumaluminate", s. allgemeine Formel II). Die Mehrzahl dieser Verbindungen besitzt eine Schichtstruktur (typischer Vertreter: Hydrotalkit), jedoch sind auch Mischhydroxide mit davon abweichender Struktur bekannt.

In der Literatur finden sich für Mischhydroxide mit Schichtstruktur eine Anzahl von synonymen Bezeichnungen, z.B. "Anionische Tone", "Anionic clays", "Hydrotalkit-ähnliche Verbindungen", "Layered double hydroxides (= LDHs)", "Feitknecht-Verbindungen" oder "Doppelschichtstrukturen".

Die Substanzklasse der Mischhydroxide mit Schichtstruktur ist in den Übersichtsartikeln von Cavani et al. oder Allmann ausführlich beschrieben (F. Cavani, F. Trifirò und A. Vaccari, "Hydrotalcitetype anionic clays: preparation, properties and applications", Catalysis Today, 11 (1991) 173-301; R. Allmann, "Doppelschichtstrukturen mit brucitähnlichen Schichtionen", Chimia 24 (1970) 99-108).

Als Beispiele für aus Polykationen aufgebaute Mischhydroxide, welche keine Schichtstruktur besitzen, seien die Ettringite genannt. Diese Verbindungen sind von Feitknecht et al. beschrieben worden, ihre Struktur wurde von Moore et al. bestimmt (W. Feitknecht und H.W. Buser, "Zur Kenntnis der nadeligen Calcium-Aluminiumhydroxysalze", Helv. Chim. Acta 32 (1949) 2298-2305; A.E. Moore und H.F. Taylor, "Crystal structure of ettringite", Acta Cryst. B 26 (1970) 386-393).

Charakteristisch für alle erfindungsgemäß verwendeten Mischhydroxide ist eine Struktur, welche aus positiv geladenen Mischhydroxid-Einheiten (Polykationen) besteht, zwischen denen sich zum Ladungsausgleich Anionen befinden. Zwischen den positiv geladenen Einheiten befinden sich neben den Anionen A in der Regel auch Lösungsmittelmoleküle L. Diese Polykationen sind in der Regel Schichten, können aber auch, z.B. beim Ettringit, andere Formen annehmen. Die Anionen zwischen den Polykationen lassen sich im allgemeinen austauschen.

Die Struktur der Mischydroxide, insbesondere der Abstand der Polykationen voneinander, kann durch Röntgendiffraktometrie ermittelt werden. Die Abstände der Polykationen voneinander, z.B. die Schichtabstände beim Hydrotalkit, sind hauptsächlich von der Natur der Anionen A abhängig und können zwischen ca. 4 Å und ca. 45 Å betragen.

Ein wichtiger Vertreter der Mischhydroxide mit Schichtstruktur ist der Hydrotalkit. Der natürlich vorkommende Hydrotalkit besitzt die chemische Zusammensetzung [Mg₆Al₂(OH)₁₆]CO₃·4H₂O. Hydrotalkit besitzt eine Struktur, in welcher Brucit-ähnliche Schichten aufgrund der Substitution einiger zweiwertiger Magnesiumionen durch dreiwertige Aluminiumionen eine positive Ladung tragen. Diese Polykationen-Schichten wechseln ab mit Zwischenschichten, welche Carbonat-Anionen sowie Wasser enthalten. Die Struktur des Hydrotalkits ist ausführlich in der oben zitierten Literatur, z.B. von Cavani et al., beschrieben.

Es sind auch einfache Hydroxid-Salze bekannt, welche aus Polykationen und dazwischen befindlichen Anionen aufgebaut sind. Während die oben beschriebenen Mischhydroxide aus Heteropolykationen aufgebaut sind, beinhaltet diese Struktur derartiger Verbindungen Isopolykationen. Beispiel dafür ist der Hydrozinkit (basisches Zinkcarbonat), dessen Struktur von Ghose bestimmt wurde (S. Ghose, "The crystal structure of hydrozincite, Zn₅(OH)₆(CO₃)₂" Acta Cryst. 17 (1964) 1051-1057). Verbindungen dieser Klasse können ebenfalls als Ausgangsmaterial für Alkoxylierungskatalysatoren dienen.

Beim Erwärmen der Mischhydroxide wird bis zu Temperaturen von ca. 200°C zunächst das zwischen den Polykationen befindliche Lösungsmittel, meist Kristallwasser, abgegeben. Bei höheren Temperaturen, etwa beim Kalzinieren auf 500°C, werden unter Zerstörung der Struktur die Polykationen abgebaut und gegebenenfalls auch das Anion zersetzt. Die Kalzinierung ist, sofern sie nicht bei allzu hohen Temperaturen durchgeführt wurde, reversibel (sog. "Memory-Effekt").

Mischhydroxide können relativ einfach im Labor hergestellt werden. Außerdem kommen eine Reihe dieser Verbindungen in der Natur als Mineralien vor; Beispiele hierfür sind:

| | |
|---|---|
| Hydrotalkit | [Mg₆Al₂(OH)₁₆]CO₃·4H₂O |
| | |
| Takovit | [Ni₆Al₂(OH)₆]CO₃·4H₂O |
| | |
| Hydrocalumit | [Ca₂Al(OH)₆]OH·6H₂O |
| | |
| Magaldrat | [Mg₁₀Al₅(OH)₃₁] (S0₄)₂·mH₂O |
| | |
| Pyroaurit | [Mg₆Fe₂(OH)₁₆]CO₃·4,5H₂O |
| | |
| Ettringit | [Ca₆Al₂(OH)₁₂](SO₄)₃·26H₂O |

Die Synthese eines Mischhydroxids im Labor geschieht in der Regel durch Fällung, wobei eine Lösung, welche die Kationen in einer gelösten Form enthält, mit einer zweiten alkalischen Lösung, welche das oder die Anionen in einer gelösten Form enthalt, zusammengebracht wird. Die genaue Ausführung dieser Fällung (pH-Wert, Temperatur, Alterung des Niederschlags) kann einen Einfluß auf die chemische Zusammensetzung der gefällten Verbindung und/oder deren Kristallitmorphologie haben.

Für das erfindungsgemäße Alkoxylierungsverfahren werden solche Mischhydroxide der allgemeinen Formel I bevorzugt, in der M(II) Magnesium, M(III) Aluminium und A Carbonat bedeutet. Insbesondere eignet sich natürlich vorkommender oder synthetisch hergestellter Hydrotalkit.

Zur erfindungsgemäßen Modifizierung ("Hydrophobierung") der beschriebenen Mischhydroxide eignen sich folgende Substanzklassen:
(a) aromatische oder heteroaromatische Mono- oder Polycarbonsäuren, insbesondere Mono- oder Dicarbonsäuren, oder deren Salze, vor allem Benzolmono- oder -dicarbonsäuren, bei denen der Benzolkern noch zusätzlich durch ein bis drei C₁- bis C₄-Alkylgruppen substituiert sein kann, z.B. Benzoesäure, p-, m- oder p-Methylbenzoesäure, p-tert.-Butylbenzoesäure, p-iso-Propylbenzoesäure, p-Ethylbenzoesäure, Phthalsäure, Isophthalsäure, Terephthalsäure, Pyridinmono- und -dicarbonsäuren sowie die Natrium-, Kalium- und Ammoniumsalze dieser Säuren;
(b) aliphatische Monocarbonsäuren oder deren Salze aus der Gruppe Phenyl-C₂- bis -C₄-alkansäuren wie Phenylessigsäure, 3-Phenylpropionsäure oder 4-Phenylbuttersäure, Cyclopentancarbonsäure, Cyclohexyl-C₂- bis -C₄-alkansäuren wie Cyclohexylessigsäure, 3-(Cyclohexyl)propionsäure oder 4-(Cyclohexyl)buttersäure, Zimtsäure, o- oder p-Chlorzimtsäure, Pyridylessigsäuren sowie die Natrium-, Kalium- und Ammoniumsalze dieser Säuren;
(c) Halbester von aliphatischen und aromatischen Dicarbonsäuren und deren Salze, vorzugsweise Phthalsäure-, Hexahydrophthalsäure-, Maleinsäure- oder Terephthalsäure-C₁- bis -C₁₈-alkylhalbester, z.B. Phthalsäure- oder Terephthalsäure-monomethylester, -monoethylester oder -monobutylester;
(d) aliphatische und vor allem alicyclische und aromatische Carbonsäureanhydride, z.B. Maleinsäureanhydrid, Phthalsäureanhydrid oder Hexahydrophthalsäureanhydrid;
(e) aliphatische oder aromatische Sulfonsäuren und deren Salze, z.B. Methansulfonsäure, Benzolsulfonsäure, p-Toluolsulfonsäure, p-Dodecylbenzolsulfonsäure, Naphthalinsulfonsäuren, Naphtholsulfonsäuren sowie die Natrium-, Kalium- und Ammoniumsalze dieser Säuren;
(f) C₈- bis C₁₈-Alkylsulfate, insbesondere Alkalimetall-C₈- bis C₁₈-Alkylsulfate, z.B. Natrium- oder Kalium-Decylsulfat oder -Dodecylsulfat;
(g) C₈- bis C₅₀-n-Alkane, vor allem C₁₀- bis C₃₀-n-Alkane wie n-Decan oder n-Dodecan;
(h) Polyetherole oder Polyetherpolyole, d.h. Ethersauerstoffbrücken aufweisende einwertige (Polyetherole, Polyalkylenglykolmonoether oder -ester) oder mehrwertige Alkohole (Polyetherpolyole), insbesondere alkoxylierte C₁- bis C₃₀-Alkohole, alkoxylierte C₁ bis C₃₀-Carbonsäuren aliphatischer oder aromatischer Struktur oder alkoxylierte C₆- bis C₃₀-Phenole, wobei pro Mol Hydroxylgruppe 1 bis 50, vor allem 2 bis 30 mol eines C₂- bis C₄-Alkylenoxids oder eine Mischung hieraus, welche im alkoxylierten Produkt zu einer statistischen Mischung oder vorzugsweise zu einer blockweisen Gruppierung der verschiedenen Alkylenoxid-Einheiten führt, eingesetzt werden; die Additive (h) sind teilweise strukturgleich mit den bei der erfindungsgemäßen Alkoxylierung von Verbindungen mit aktiven Wasserstoffatomen unter Verwendung der entsprechend additivierten Mischhydroxide I oder II erhaltenen Produkten;
(j) Alkohole oder Phenole, welche nicht zwischen den Schichten des Mischhydroxids I oder II eingebaut sind (sofern dieses Mischhydroxid Schichtstruktur aufweist), insbesondere aliphatische oder cycloaliphatische C₁- bis C₃₀-Alkohole oder C₆- bis C₃₀-Phenole, vor allem n-Alkanole (Fettalkohole) mit 9 bis 18 C-Atomen, z.B. n-Decanol, n-Dodecanol, n-Tetradecanol, n-Hexadecanol oder n-Octadecanol; die Additive (j) sind teilweise strukturgleich mit den bei der erfindungsgemäßen Alkoxylierung unter Verwendung der entsprechend additivierten Mischhydroxide I oder II als Ausgangsmaterialien eingesetzten Verbindungen mit aktiven Wasserstoffatomen wie den Verbindungen III.

Es können auch Gemische der genannten Additive aus einer der Klassen (a) bis (j) oder aus verschiedenen Klassen eingesetzt werden.

Die Halbester (c) können vorteilhaft durch Umsetzung der entsprechenden Carbonsäureanhydride, beispielsweise der Anhydride (d), mit Alkoholen wie C₁- bis C₁₈-Alkanolen erhalten werden. Als Alkohol-Komponente können auch Gemische eingesetzt werden. Als Anhydrid-Komponente eignet sich beispielsweise Phthalsäureanhydrid, Hexahydrophthalsäureanhydrid oder Maleinsäureanhydrid.

Dabei ist es auch möglich, die Alkohol-Komponente im Überschuß einzusetzen, so daß sie gleichzeitig als Lösungsmittel bei der Modifizierung der Mischhydroxide dient. Falls bei der Alkoxylierung ein Alkohol als Wasserstoff-aktive Verbindung dient, wird am besten dieser Alkohol für die Umsetzung mit Anhydriden gewählt, so daß ein aufwendiges Filtrieren und Trocknen entfallen kann.

In einer bevorzugten Ausführungsform verwendet man beim erfindungsgemäßen Alkoxylierungsverfahren ein Mischhydroxid I oder II, welches als Additive Benzolmono- oder -dicarbonsäuren, bei denen der Benzolkern noch zusätzlich durch ein bis drei C₁- bis C₄-Alkylgruppen substituiert sein kann, Phenyl-C₂- bis -C₄-alkansäuren, Cyclohexancarbonsäure, Cyclohexyl-C₂- bis -C₄-alkansäuren, Phthalsäure-, Hexahydrophthalsäure-, Maleinsäure- oder Terephthalsäure-C₁- bis -C₁₈-alkylhalbester, Maleinsäureanhydrid, Phthalsäureanhydrid, Hexahydrophthalsäureanhydrid, Benzolsulfonsäure, p-Toluolsulfonsäure, p-Dodecylbenzolsulfonsäure, Alkalimetall-Dodecylsulfate, n-Dodecan, n-Alkanole mit 9 bis 18 C-Atomen oder Salze der genannten Säuren.

Ganz besonders bevorzugt werden als Additiv Benzoesäure und ihre Alkalimetallsalze wegen der hervorragenden Eigenschaften als Alkoxylierungskatalysatoren und der Wirtschaftlichkeit dieser Verbindungen, d.h. ihrer leichten und preiswerten Zugänglichkeit.

Das molare Verhältnis der Additive (a) bis (j) zu den Mischhydroxiden I oder II beträgt üblicherweise 0,01:1 bis 10:1, vor allem 0,05:1 bis 5:1, insbesondere 0,2:1 bis 2:1.

Die Struktur der Mischhydroxide bleibt nach der Modifizierung mit den Additiven (a) bis (j) im wesentlichen erhalten; insbesondere bleiben die Polykationen im wesentlichen unverändert. Die Abstände der Polykationen voneinander können bei der Modifizierung mehr oder weniger stark verändert werden. In einigen Fällen befinden sich die Additive nach der Modifizierung in neutraler oder anionischer Form zwischen den Polykationen der Mischhydroxide, wobei sie die dort befindlichen Anionen ganz oder teilweise verdrängen können. In bestimmten Fällen kann die Modifizierung der Mischhydroxide auch als eine Art Austausch der ursprünglich vorhandenen Anionen gegen Additiv-Anionen bzw. aus dem Additiv gebildete Anionen angesehen werden. Die Einlagerung der Additive zwischen die Polykationen der Mischhydroxide zeigt sich in der Regel durch eine Aufweitung des Abstands zwischen den Polykationen und kann durch Röntgendiffraktometrie nachgewiesen werden.

Eine derartige Modifizierung kann zum Zwecke der Hydrophobierung, der Veränderung der Dispergierbarkeit, der Veränderung der katalytischen Eigenschaften und/oder der Veränderung der rheologischen Eigenschaften der Mischhydroxide erfolgen. In anderen Fällen bewirkt die Modifizierung lediglich eine Beschichtung der Mischhydroxide auf deren Oberfläche, wobei der Abstand der Polykationen unverändert bleibt. Eine derartige oberflächliche Beschichtung wird auch als "coating" bezeichnet und kann z.B. auch zur Hydrophobierung der Mischhydroxide dienen.

Als Verbindung mit aktiven Wasserstoffatomen können alle Verbindungen eingesetzt werden, die ein oder mehrere acide Wasserstoffatome aufweisen, welche mit Alkylenoxiden reagieren können. Insbesondere sind hierbei Alkohole, Phenole, Kohlenhydrate, Carbonsäuren, Carbonsäureamide, Amine und Mercaptane zu nennen, welche unter die allgemeine Formel III 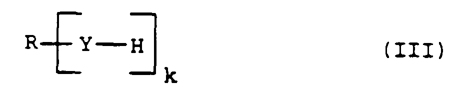 fallen, in der R einen üblichen Kohlenwasserstoffrest bezeichnet, der noch Heteroatome enthalten und weitere funktionelle Gruppen tragen kann, Y für O, S, NH oder NR steht und k 1, 2 oder 3 bedeutet.

Je nach Wert von k können die Reste R mono-, di- oder trivalent sein. Bevorzugt werden mono- und divalente Reste, die größte Bedeutung kommt monovalenten Resten zu.

Als Reste R sind vor allem zu nennen:
- geradkettige oder verzweigte C₁-C₃₀-Alkylgruppen und C₃-C₃₀-Alkenylgruppen, welche durch ein oder mehrere nicht benachbarte Sauerstoffatome unterbrochen sein und zusätzliche Hydroxylgruppen tragen können, z.B. Methyl, Ethyl, n-Propyl, iso-Propyl, n-Butyl, 2-Ethylhexyl, n-Octyl, iso-Nonyl, n-Decyl, n-Dodecyl, iso-Tridecyl, Myristyl, Cetyl, Stearyl, Eicosyl, 2-Propenyl, Oleyl, Linolyl, Linolenyl, 2-Methoxyethyl, 2-Ethoxyethyl, 2-Butoxyethyl, 4-Methoxybutyl, 4-(4'-Methoxybutyloxy)butyl, 2-Hydroxyethyl oder 4-Hydroxybutyl;
- C₁-C₃₀-Acylgruppen, welche zusätzlich Hydroxylgruppen tragen können, z.B. Formyl, Acetyl, Propionyl, Butyryl, Valeryl, Decanoyl, Lauroyl, Myristoyl, Palmitoyl, Stearoyl oder Lactyl;
- monovalente Kohlenhydrat-Reste von Mono- oder Disacchariden für den Fall Y=O, z.B. von Glucose, Mannose, Fructose, Saccharose, Lactose oder Maltose;
- Arylgruppen mit insgesamt 6 bis 20 C-Atomen, welche zusätzlich durch C₁-C₄-Alkylgruppen, Hydroxylgruppen, C₁-C₄-Alkoxygruppen und Aminogruppen substituiert sein können, z.B. Phenyl, Tolyl, Xylyl, Hydroxyphenyl, Methoxyphenyl, Aminophenyl oder Naphthyl;
- Arylcarbonylgruppen mit insgesamt 7 bis 21 C-Atomen, welche zusätzlich durch C₁-C₄-Alkylgruppen, Hydroxylgruppen, C₁-C₄-Alkoxygruppen und Aminogruppen substituiert sein können, z.B. Benzoyl;
- divalente Reste, die sich beispielsweise für den Fall Y=O von Diolen, Dihydroxyaromaten oder Bisphenolen ableiten, wie 1,2-Ethylen, 1,3-Propylen, 1,4-Butylen, Phenylen oder der Rest von Bisphenol A;
- trivalente Reste, die sich beispielsweise für den Fall Y=O von Triolen wie Glycerin ableiten;
- polyvalente Reste, die sich insbesondere von Polyolen (Y=O), z.B. Pentaerythrit, oder von Kohlenhydraten ableiten.

In einer bevorzugten Ausführungsform werden als wasserstoffaktive Verbindungen III C₁-C₃₀-Alkanole und C₃- bis C₃₀-Alkenole, insbesondere C₈- bis C₃₀-Alkanole und -Alkenole (Fettalkohole) eingesetzt.

Als C₂- bis C₄-Alkylenoxide kommen vor allem 1,2-Propylenoxid, 1,2-Butylenoxid, 2,3-Butylenoxid und insbesondere Ethylenoxid in Betracht.

Die beschriebenen modifizierten Mischhydroxide werden bei den Alkoxylierungsreaktionen in der Regel in Mengen von 0,1 bis 5 Gew.-%, vorzugsweise 0,15 bis 2 Gew.-%, besonders bevorzugt 0,2 bis 0,8 Gew.-%, bezogen auf das Gewicht der Verbindungen mit aktiven Wasserstoffatomen, eingesetzt.

Die Alkoxylierungsreaktionen werden üblicherweise bei 80 bis 230°C, vorzugsweise bei 100 bis 200°C, besonders bevorzugt bei 160 bis 180°C durchgeführt. Die Reaktion wird in der Regel unter Druck durchgeführt, beispielsweise arbeitet man in einem Autoklaven bei 2 bis 10 bar, insbesondere 3 bis 7 bar. Man führt die Umsetzung vorteilhafterweise ohne Lösungsmittel durch; ein inertes Lösungsmittel kann aber zugegeben sein.

Ein Teil der Mischhydroxide I und II stellt neue Verbindungen dar. Daher sind auch Gegenstand der vorliegenden Erfindung aus Polykationen aufgebaute Mischhydroxide der allgemeinen Formel Ia und IIa

[M(II)₁₋ₓM(III)ₓ(OH)₂]A_{x/n} · m L (Ia)

[LiAl₂(OH)₆]A_{1/n} · m L (IIa)

in denen
- M(II): mindestens ein zweiwertiges Metallion,
- M(III): mindestens ein dreiwertiges Metallion,
- A: mindestens ein anorganisches Anion und
- L: ein organisches Lösungsmittel oder Wasser bedeutet,
- n: die Wertigkeit des anorganischen Anions A oder bei mehreren Anionen A deren mittlere Wertigkeit bezeichnet und
- x: einen Wert von 0,1 bis 0,5 und
- m: einen Wert von 0 bis 10 annehmen kann,
welche mit
(a) aromatischen oder heteroaromatischen Mono- oder Polycarbonsäuren oder deren Salzen,
(b) aliphatischen Mono- oder Polycarbonsäuren oder deren Salzen aus der Gruppe Phenyl-C₂- bis C₄-alkansäuren, Cyclopentancarbonsäure, Cyclohexancarbonsäure, Cyclohexyl-C₂- bis C₄-alkansäuren, Zimtsäure, o- oder p-Chlorzimtsäure und Pyridylessigsäuren,
(c) Halbestern von Dicarbonsäuren oder deren Salzen,
(d) Maleinsäureanhydrid, Phthalsäureanhydrid oder Hexahydrophthalsäureanhydrid,
(e) aliphatischen Sulfonsäuren oder deren Salzen,
(f) C₈- bis C₁₈-Alkylsulfaten,
(g) C₈- bis C₅₀-n-Alkanen oder
(j) n-Alkanolen mit 9 bis 18 C-Atomen oder Phenolen, welche nicht zwischen den Schichten des Mischhydroxids Ia oder IIa eingebaut sind,

als Additiven modfiziert sind,
mit Ausnahme von Benzoesäure und seinen Salzen im Falle von Lithium-Aluminium-Mischhydroxid mit Schichtstruktur,
mit Ausnahme von Terephthalsäure, p- und o-Chlorzimtsäure und den entsprechenden Salzen und von n-Dodecylsulfat im Falle von Magnesium-Aluminium-Mischhydroxiden mit Schichtstruktur sowie mit Ausnahme von Phthalsäure, Isophthalsäure, Terephthalsäure und Pyridindicarbonsäuren im Falle von Calcium-Aluminium-Mischhydroxiden.

Aus der Literaturstelle P.K. Dutta und M. Puri, J. Phys. Chem. 1989, 93, 376-381 (4) ist ein Lithium-Aluminium-Mischhydroxid mit Schichtstruktur mit eingelagerten Benzoat-Anionen bekannt.

K. Chibwe und W. Jones beschreiben in der Literaturstelle J. Chem. Soc., Chem. Commun. 1989, 926-927 (5) synthetischen Hydrotalkit mit eingelagertem Natrium-Dodecylsulfat und p-Toluolsulfonsäure.

In der Literaturstelle K. Chibwe, J.B. Valim und W. Jones, Prep. Am. Chem. Soc. 34 (1989) 507-510 (6) werden Magnesium-Aluminium-Mischhydroxide mit Schichtstruktur mit eingelagerter Terephthalsäure, p-Toluolsulfonsäure, 2-Naphthol-6-sulfonsäure, p- oder o-Chlorzimtsäure und n-Dodecylsulfat offenbart.

In allen drei Literaturstellen (4) bis (6) werden lediglich Anionenaustauscheigenschaften dieser Mischhydroxide untersucht.

Aus der DE-A 40 02 988 (7) und der DE-A 41 06 404 (8) sind Calcium-Aluminium-Mischhydroxide bekannt, die u.a. mit Phthalsäure, Isophthalsäure, Terephthalsäure oder Pyridincarbonsäuren modifiziert sind. Diese Verbindungen werden als Stabilisatoren für halogenhaltige thermoplastische Harze wie PVC empfohlen.

Die Herstellung der erfindungsgemäßen Mischhydroxide Ia und IIa, aber auch der restlichen Mischhydroxide I und II, d.h. die Modifizierung mit den Additiven (a) bis (j), kann in vorteilhafter Weise auf drei verschiedenen Wegen erfolgen:
(i) die Verbindungen Ia oder IIa werden durch direkte Synthese ("in situ") aus wäßrigen Lösungen von M(II)- und M(III)-Salzen bzw. wäßrigen Lösungen von Lithium- und Aluminiumsalzen alkalisch in Gegenwart der Additive (a) bis (g) und (j) gefällt;
(ii) die nicht modifizierten Mischhydroxide werden mit den Additiven (a) bis (j) bzw. (a) bis (g) und (j) in Gegenwart oder in Abwesenheit eines Lösungsmittels bei Temperaturen von 0 bis 200°C umgesetzt;
(iii) die nicht modifizierten Mischhydroxide werden auf Temperaturen > 200°C erwärmt, wobei die Temperatur nur so hoch gewählt werden darf, daß das Mischhydroxid anschließend wieder vollständig in die ursprüngliche Kristallstruktur zurückkehren kann, das so behandelte Mischhydroxid wird mit den Additiven (a) bis (j) bzw. (a) bis (g) und (j) umgesetzt und anschließend gegebenenfalls mit Wasser nachbehandelt.

Bei (ii) geschieht die Umsetzung in der Regel durch Mischen der Komponenten in einer geeigneten Apparatur, z.B. einem Rührkessel, Mischer, Kneter oder einer Kugelmühle vorzugsweise bei leicht erhöhter Temperatur, etwa bei 60° bis 200°C. Man arbeitet üblicherweise bei Drücken von 0,1 bis 50 bar, vorzugsweise 0,5 bis 20 bar. Falls ein Lösungsmittel anwesend sein soll, haben sich hierbei Wasser, Aceton oder ein Alkohol wie Methanol, Ethanol oder Isopropanol bewährt. Der Umsetzung kann sich eine Nachbehandlung mit Lösungsmittel und/oder eine Trocknung anschließen.

Bei (iii) geschieht die umsetzung in der Regel durch Mischen der Komponenten in einer geeigneten Apparatur, z.B. einem Mischer, Kneter oder einer Kugelmühle bei stark erhöhter Temperatur, etwa bei 250 bis 550°C. Der Umsetzung kann sich eine Nachbehandlung mit Lösungsmittel und/oder eine Trocknung anschließen. Durch die Temperaturbehandlung wird zunächst die Struktur des Mischhydroxids zumindest teilweise zerstört. Nach der Behandlung mit dem Additiv wird die ursprüngliche Struktur des Mischhydroxids wieder erhalten, insbesondere, wenn eine Nachbehandlung mit Wasser stattfindet.

Die mit den Additiven (a) bis (j) modifizierten Mischhydroxide I und II können hervorragend zur Alkoxylierung von Verbindungen mit aktiven Wasserstoffatomen eingesetzt werden. Sie haben den Vorteil, daß sie eine hohe Selektivität und eine hohe Reaktivität aufweisen und damit sehr kurze Reaktions- und Cycluszeiten bei der Alkoxylierung auftreten. Zudem ist das Nebenproduktspektrum sehr gering, insbesondere erhält man geringe Polyalkylenglykol-Werte. Die hergestellten Alkoxylierungsprodukte weisen somit eine sehr enge Homologenverteilung auf. Derartige Alkoholalkoxylate, besonders Alkoholethoxylate, haben gegenüber den breiter-verteilten Produkten, meist hergestellt mit NaOH, KOH oder Natriummethylat, anwendungstechnische Vorteile. So zeigen sie z.B. niedrigere Fließpunkte, geringere Grenzflächenspannungen, bessere Wasserlöslichkeit und - durch die Verringerung des freien Alkohols (der nicht alkoxylierten Ausgangsverbindung) bedingt - einen besseren Geruch. Der Vorteil einer engeren Homologenverteilung tritt auch deutlich gegenüber den aus dem Stand der Techniken bekannten mit aliphatischen Mono- oder Dicarbonsäuren modifizierten Mischhydroxiden auf.

Die modifizierten Mischhydroxide I und II haben bei der Alkoxylierung den Vorteil, daß sie sich besser in das Reaktionsmedium, z.B. langkettige Fettalkohole, dispergieren lassen, jedoch nach der Reaktion durch Filtration leicht wieder entfernt werden können. Falls für eine weitere Anwendung der Produkte nicht hinderlich, können die Mischhydroxide auch im Produkt verbleiben, wo sie eine Änderung dessen rheologischer Eigenschaften bewirken können.

Nach erfolgtem Einsatz sind die modifizierten Mischhydroxide I und II im Prinzip wiederverwendbar, nachdem sie in geeigneter Weise aus der Reaktionsmischung abgetrennt wurden.

### Beispiele

### Herstellung von mit Additiven modifizierten Mischhydroxiden

Die in Tabelle 1 angegebenen modifizierten Mischhydroxide als Beispiele 1 bis 13 und Vergleichsbeispiele 14 und 15 wurden gemäß einer der Herstellungsvorschrift A bis E dargestellt.

**Tabelle 1**

| Bsp. Nr. | Mischhydroxid | Additiv | Additiv-Menge [g] | Herstellungsvorschrift |
|---|---|---|---|---|
| 1 | Hydrotalkit | Benzoesäure | 150 | A |
| 2 | Hydrotalkit | p-tert.-Butylbenzoesäure | 200 | A |
| 3 | Hydrotalkit | Benzoesäure | 80 | B |
| 4 | Hydrotalkit | Benzoesäure | 40 | B |
| 5 | Hydrotalkit | Benzoesäure | 20 | B |
| 6 | Hydrotalkit | p-tert.-Butylbenzoesäure | 100 | B |
| 7 | Zink-Aluminium-Mischhydroxid | Benzoesäure | 80 | B |
| 8 | Hydrotalkit | Phthalsäureanhydrid | 75 | C |
| 9 | Hydrotalkit | Benzoesäure | 80 | C |
| 10 | Hydrotalkit | Benzoesäure | 150 | D |
| 11 | Hydrotalkit | p-tert.-Butylbenzoesäure | 200 | D |
| 12 | Hydrotalkit | p-Methylbenzoesaure | 200 | D |
| 13 | Calcium-Aluminium-Mischhydroxid | Phthalsäure | 33 | E |

| zum Vergleich: | | | | |
|---|---|---|---|---|
| 14 | Hydrotalkit | Laurinsäure | 250 | A |
| 15 | Hydrotalkit | Laurinsäure | 200 | D |

Als Hydrotalkit wurde handelsübliches Material eingesetzt.

Das Zink-Aluminium-Mischhydroxid aus Beispiel 7 wurde folgendermaßen hergestellt:

Zu 500 ml 2-molarer Natriumnitrat-Lösung wurden unter Rühren bei 80°C gleichzeitig eine Lösung von 0,2 mol Aluminiumnitrat und 0,7 mol Zinknitrat in 1 Liter Wasser und eine Lösung von 1 mol Natriumcarbonat in 1 Liter Wasser gegeben. Der Zulauf der beiden Lösungen erfolgte innerhalb von 60 Minuten und wurde so zudosiert, daß die Mischung stets einen pH-Wert von 6,5 besaß. Nach Beendigung der Fällung wurde die Mischung weitere 30 Minuten bei 80°C gerührt, wobei wiederum der pH-Wert konstant gehalten wurde. Der Niederschlag wurde abfiltriert, nitratfrei gewaschen und bei 100°C getrocknet. Aus einer Elementaranalyse ergab sich folgende ungefähre chemische Zusammensetzung des Zink-Aluminium-Mischhydroxids: [Zn₆Al₂(OH)₁₆]CO₃·mH₂O.

### Herstellungsvorschrift A:

300 g Mischhydroxid wurden zusammen mit einer gesättigten Lösung des Additivs in Isopropanol in einen Rührbehälter gefüllt. Die Mischung wurde unter Rühren zwei Stunden lang unter Rückfluß gekocht. Anschließend wurde die feste Phase abfiltriert, mit reinem Isopropanol gewaschen und bei 110°C getrocknet.

### Herstellungsvorschrift B:

150 g Mischhydroxid wurden zusammen mit dem Additiv in einen Kneter gegeben. Die Mischung wurde zwei Stunden lang bei 80°C geknetet.

### Herstellungsvorschrift C:

150 g Mischhydroxid wurden zusammen mit dem Additiv sowie 100 g Dodekanol als Lösungsmittel in einen Kneter gegeben. Die Mischung wurde zwei Stunden lang bei 80°C geknetet.

### Herstellungsvorschrift D:

300 g Mischhydroxid wurden zwei Stunden lang bei 500°C getempert und anschließend zusammen mit einer gesättigten Lösung des Additivs in Wasser in einen Rührbehälter gefüllt. Die Mischung wurde unter Rühren zwei Stunden lang auf 80°C erwärmt. Anschließend wurde die feste Phase abfiltriert, mit reinem Wasser gewaschen und bei 110°C getrocknet.

### Herstellungsvorschrift E:

22 g Calciumhydroxid wurden zusammen mit 8 g Natriumhydroxid und 16 g Aluminiumhydroxid in 500 ml Wasser suspendiert und auf 80°C erwärmt. Anschließend wurde bei dieser Temperatur eine Lösung aus 33 g Phthalsäure in 400 ml Wasser unter Rühren zugegeben. Die Suspension wurde weitere 2 Stunden bei 80°C gerührt. Der Feststoff wurde abfiltriert, mit Wasser gewaschen und bei 110°C getrocknet.

Weiterhin wurden folgende erfindungsgemäße und modifizierte Mischhydroxide aus handelsüblichem Hydrotalkit hergestellt:

### Beispiel 16 (Hydrotalkit + Benzoesäure)

10 g getrockneter Hydrotalkit wurden zusammen mit 4 g Benzoesäure in einem Autoklaven unter Rühren auf 150°C aufgeheizt. Dabei stieg der Druck um ca. 5 bar. Der Autoklav wurde entspannt und die Mischung weitere vier Stunden bei 150°C behandelt. Anschließend wurde das Produkt eine Stunde lang bei 200°C im Trockenschrank behandelt.

### Beispiel 17 (Hydrotalkit + n-Decanol)

10 g getrockneter Hydrotalkit wurden in 200 g n-Decanol bei 60°C 30 Minuten lang mittels eines Intensivrührers dispergiert. Anschließend wurde der Feststoff abfiltriert.

### Beispiel 18 (Hydrotalkit + Benzoesäure)

900 g getrockneter Hydrotalkit wurden zusammen mit 90 g Benzoesäure in einem 5-Liter-Pflugscharmischer bei 80°C vier Stunden lang behandelt. Der Mischer lief mit einer Drehzahl von 140 U/Minute. Anschließend wurde das Produkt bei 150°C eine Stunde lang im Trockenschrank behandelt.

### Ethoxylierungen mit den modifizierten Mischhydroxiden

### Allgemeine Arbeitsvorschrift für die Ethoxylierung:

1 mol Dodecanol (186 g) wurde mit 0,5 Gew.-% Katalysator aus den Beispielen 1 bis 12 bzw. den Vergleichsbeispielen 14 oder 15 in einem Stahlautoklaven vorgelegt und bei 120°C 1 Stunde lang unter Vakuum entwässert. Anschließend wurde die Temperatur auf 1700C erhöht und bei dieser Temperatur wurden 5 mol Ethylenoxid (220 g) aufgepreßt. Dabei wurde die Ethylenoxid-Zufuhr so geregelt, daß der Autoklaveninnendruck nicht Über 6 bar stieg. Nach beendeter Ethylenoxid-Zufuhr wurde bei 170°C bis zur Druckkonstanz nachgerührt. Das erhaltene Produkt wurde heiß filtriert.

In Tabelle 2 sind Reaktionszeiten, Polyethylenglykol-Werte (bezogen auf PEG 400), Restalkoholgehalte und Homologenverteilungen angegeben. Letztere sind mittels Gaschromatographie ermittelt worden und in Flächenprozent angegeben.

Man erkennt aus den Beispielen, daß im Vergleich zum Stand der Technik (Vergleichsbeispiel 14 und 15) eine Verbesserung bezuglich Polyethylenglykol-Wert und Maximum in der Homologenverteilung erreicht wurde. Die Restalkoholgehalte sind in vielen Fällen niedriger als bei den Vergleichsbeispielen.

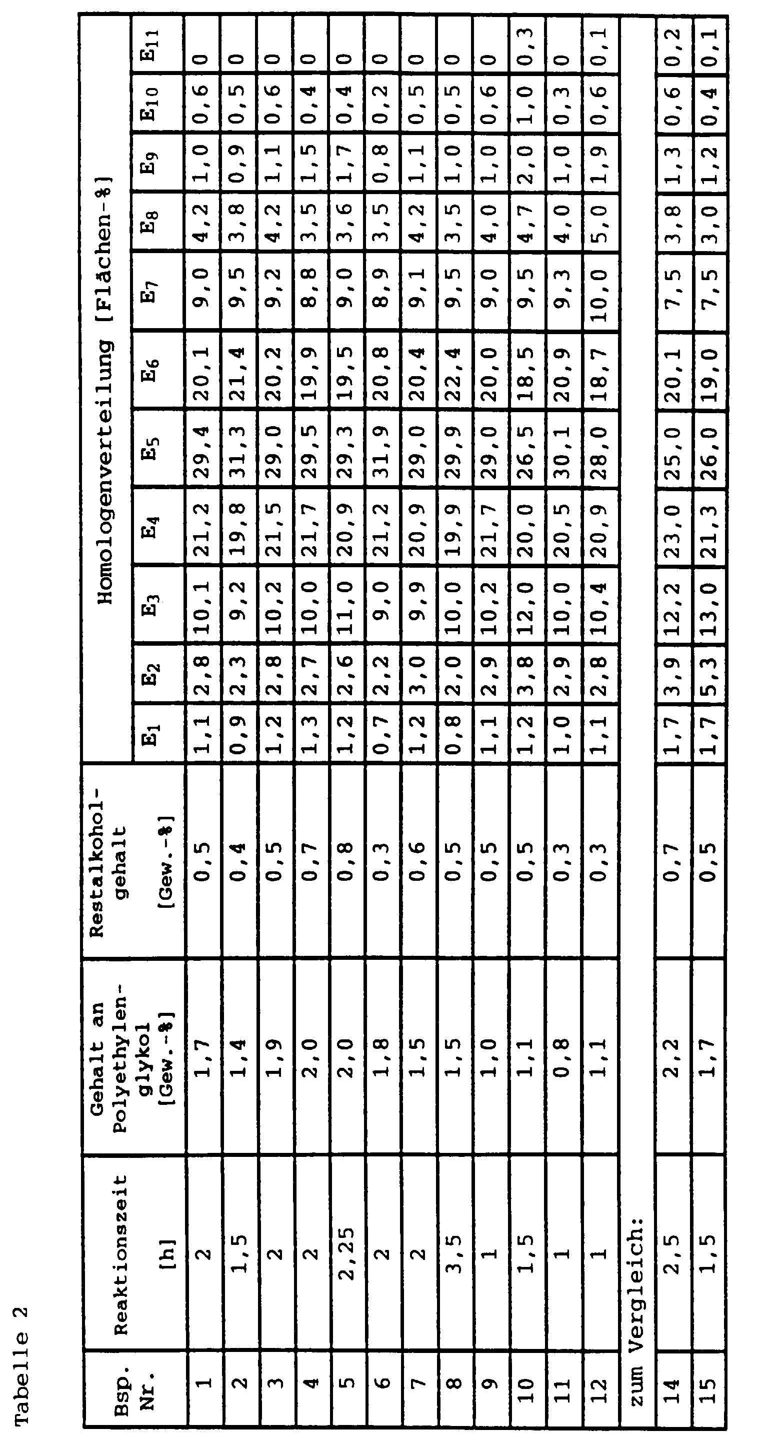

## Patentansprüche

1. Verfahren zur Herstellung von Alkoxylierungsprodukten durch Umsetzung von Verbindungen mit aktiven Wasserstoffatomen mit C₂- bis C₄-Alkylenoxiden in Gegenwart eines mit Additiven modifizierten, aus Polykationen aufgebauten Mischhydroxids der allgemeinen Formel I oder II
[M(II)₁₋ₓM(III)ₓ(OH)₂]A_{x/n} · m L (I)
[LiAl₂(OH)₆]A_{1/n} · m L (II)
in denen
M(II) mindestens ein zweiwertiges Metallion,
M(III) mindestens ein dreiwertiges Metallion,
A mindestens ein anorganisches Anion und
L ein organisches Lösungsmittel oder Wasser bedeutet,
n die Wertigkeit des anorganischen Anions A oder bei mehreren Anionen A deren mittlere Wertigkeit bezeichnet und
x einen Wert von 0,1 bis 0,5 und
m einen Wert von 0 bis 10 annehmen kann,
als Alkoxylierungskatalysator, dadurch gekennzeichnet, daß das Mischhydroxid als Additive
(a) aromatische oder heteroaromatische Mono- oder Polycarbonsäuren oder deren Salze,
(b) aliphatische Mono- oder Polycarbonsäuren oder deren Salze mit einem isocyclischen oder heterocyclischen Ring in der Seitenkette,
(c) Halbester von Dicarbonsäuren oder deren Salze,
(d) Carbonsäureanhydride,
(e) aliphatische oder aromatische Sulfonsäuren oder deren Salze,
(f) C₈- bis C₁₈-Alkylsulfate,
(g) langkettige Paraffine,
(h) Polyetherole oder Polyetherpolyole oder
(j) Alkohole oder Phenole, welche nicht zwischen den Schichten des Mischhydroxids I oder II eingebaut sind,
enthält.

2. Verfahren zur Herstellung von Alkoxylierungsprodukten nach Anspruch 1 in Gegenwart eines Mischhydroxids der allgemeinen Formel I, in der M(II) Magnesium, M(III) Aluminium und A Carbonat bedeutet.

3. Verfahren zur Herstellung von Alkoxylierungsprodukten nach Anspruch 1 oder 2, dadurch gekennzeichnet, daß das Mischhydroxid I oder II als Additive Benzolmono- oder -dicarbonsäuren, bei denen der Benzolkern noch zusätzlich durch ein bis drei C₁- bis C₄-Alkylgruppen substituiert sein kann, Phenyl-C₂- bis -C₄-alkansäuren, Cyclohexancarbonsäure, Cyclohexyl-C₂- bis -C₄-alkansäuren, Phthalsäure-, Hexahydrophthalsäure-, Maleinsäure- oder Terephthalsäure-C₁- bis -C₁₈-alkylhalbester, Maleinsäureanhydrid, Phthalsäureanhydrid, Hexahydrophthalsaureanhydrid, Benzolsulfonsäure, p-Toluolsulfonsäure, p-Dodecylbenzolsulfonsäure, Alkalimetall-Dodecylsulfate, n-Dodecan, n-Alkanole mit 9 bis 18 C-Atomen oder Salze der genannten Säuren enthält.

4. Verfahren zur Herstellung von Alkoxylierungsprodukten nach den Ansprüchen 1 bis 3, dadurch gekennzeichnet, daß man die Additive (a) bis (j) im molaren Verhältnis zu den Mischhydroxiden I oder II von 0,01:1 bis 10:1 einsetzt.

5. Verfahren zur Herstellung von Alkoxylierungsprodukten nach den Ansprüchen 1 bis 4 durch Umsetzung von C₁- bis C₃₀-Alkanolen oder C₃- bis C₃₀-Alkenolen als Verbindungen mit aktiven Wasserstoffatomen.

6. Verfahren zur Herstellung von Alkoxylierungsprodukten nach den Ansprüchen 1 bis 5, dadurch gekennzeichnet, daß man die Mischydroxide I oder II in Mengen von 0,1 bis 5 Gew.-%, bezogen auf das Gewicht der Verbindungen mit aktiven Wasserstoffatomen, einsetzt.

7. Verfahren zur Herstellung von Alkoxylierungsprodukten nach den Ansprüchen 1 bis 6, dadurch gekennzeichnet, daß man die Umsetzung bei Temperaturen von 80 bis 230°C und Drücken von 2 bis 10 bar durchführt.

8. Verwendung von mit Additiven modifizierten, aus Polykationen aufgebauten Mischhydroxiden I und II gemäß Anspruch 1 als Alkoxylierungskatalysatoren bei der Umsetzung von Verbindungen mit aktiven Wasserstoffatomen mit C₂- bis C₄-Alkylenoxiden.

9. Aus Polykationen aufgebaute Mischhydroxide der allgemeinen Formel Ia und IIa
[M(II)₁₋ₓM(III)ₓ(OH)₂]A_{x/n} · m L (Ia)
[LiAl₂(OH)₆]A_{1/n} · m L (IIa)
in denen
M(II) mindestens ein zweiwertiges Metallion,
M(III) mindestens ein dreiwertiges Metallion,
A mindestens ein anorganisches Anion und
L ein organisches Lösungsmittel oder Wasser bedeutet,
n die Wertigkeit des anorganischen Anions A oder bei mehreren Anionen A deren mittlere Wertigkeit bezeichnet und
x einen Wert von 0,1 bis 0,5 und
m einen Wert von 0 bis 10 annehmen kann,
welche mit
(a) aromatischen oder heteroaromatischen Mono- oder Polycarbonsäuren oder deren Salzen,
(b) aliphatische Monocarbonsäuren oder deren Salze aus der Gruppe Phenyl-C₂-bis-C₄-alkansäuren, Cyclopentancarbonsäure, Cyclohexancarbonsäure, Cyclohexyl-C₂-bis-C₄-alkansäuren, Zimtsäure, o- oder p-Chlorzimtsäure und Pyridylessigsäuren,
(c) Halbestern von Dicarbonsäuren oder deren Salzen,
(d) Maleinsäureanhydrid, Phthalsäureanhydrid oder Hexahydrophthalsäureanhydrid,,
(e) aliphatischen Sulfonsäuren oder deren Salzen,
(f) C₈- bis C₁₈-Alkylsulfaten,
(g) C₈-bis C₅₀-n-Alkanen oder
(j) n-Alkanolen mit 9 bis 18 C-Atomen oder Phenolen, welche nicht zwischen den Schichten des Mischhydroxids Ia oder IIa eingebaut sind,
als Additiven modfiziert sind,
mit Ausnahme von Benzoesäure und seinen Salzen im Falle von Lithium-Aluminium-Mischhydroxid mit Schichtstruktur,
mit Ausnahme von Terephthalsäure, p- und o-Chlorzimtsäure und den entsprechenden Salzen und von n-Dodecylsulfat im Falle von Magnesium-Aluminium-Mischhydroxiden mit Schichtstruktur
sowie mit Ausnahme von Phthalsäure, Isophthalsäure, Terephthalsäure und Pyridindicarbonsäuren im Falle von Calcium-Aluminium-Mischhydroxiden.

10. Verfahren zur Herstellung von Mischhydroxiden Ia und IIa gemäß Anspruch 9, dadurch gekennzeichnet, daß man die Verbindungen Ia oder IIa aus wäßrigen Lösungen von M(II) - und M(III)-Salzen bzw. wäßrigen Lösungen von Lithium- und Aluminiumsalzen alkalisch in Gegenwart der Additive (a) bis (g) und (j) fällt.

11. Verfahren zur Herstellung von Mischhydroxiden Ia und IIa gemäß Anspruch 9, dadurch gekennzeichnet, daß man die nicht modifizierten Mischhydroxide mit den Additiven (a) bis (g) und (j) in Gegenwart oder in Abwesenheit eines Lösungsmittels bei Temperaturen von 0 bis 200°C umsetzt.

12. Verfahren zur Herstellung von Mischhydroxiden Ia und IIa gemäß Anspruch 9, dadurch gekennzeichnet, daß man die nicht modifizierten Mischhydroxide auf Temperaturen > 200°C erwärmt, wobei die Temperatur nur so hoch gewählt werden darf, daß das Mischhydroxid anschließend wieder vollständig in die ursprüngliche Kristallstruktur zurückkehren kann, das so behandelte Mischhydroxid mit den Additiven (a) bis (g) und (j) umsetzt und anschließend gegebenenfalls mit Wasser nachbehandelt.

## Claims

1. A process for preparing alkoxylation products by reacting compounds having active hydrogen atoms with C₂-C₄-alkylene oxides in the presence of a mixed hydroxide built up of polycations and modified with additives and having the general formula I or II
[M(II)₁₋ₓM(III)ₓ(OH)₂]A_{x/n} · m L (I)
[LiAl₂(OH)₆]A_{1/n} · m L (II)
where
M(II) is at least one divalent metal ion,
M(III) is at least one trivalent metal ion,
A is at least one inorganic anion and
L is an organic solvent or water,
n is the valence of the inorganic anion A or in the case of a plurality of anions A is their mean valence and
x can assume a value of from 0.1 to 0.5 and
m can assume a value of from 0 to 10,
as alkoxylation catalyst, wherein the mixed hydroxide contains as additives
(a) aromatic or heteroaromatic mono- or polycarboxylic acids or their salts,
(b) aliphatic mono- or polycarboxylic acids or their salts having an isocyclic or heterocyclic ring in the side chain,
(c) monoesters of dicarboxylic acids or their salts,
(d) carboxylic anhydrides,
(e) aliphatic or aromatic sulfonic acids or their salts,
(f) C₈-C₁₈-alkyl sulfates,
(g) long-chain paraffins,
(h) polyetherols or polyether polyols or
(j) alcohols or phenols, which are not built in between the layers of the mixed hydroxide I or II.

2. A process for preparing alkoxylation products as claimed in claim 1 in the presence of a mixed hydroxide of the general formula I in which M(II) is magnesium, M(III) is aluminum and A is carbonate.

3. A process for preparing alkoxylation products as claimed in claim 1 or 2, wherein the mixed hydroxide I or II contains as additives benzenemonocarboxylic or benzenedicarboxylic acids, in which the benzene ring can be additionally substituted by from one to three C₁-C₄-alkyl groups, phenyl-C₂-C₄-alkanoic acids, cyclohexanecarboxylic acid, cyclohexyl-C₂-C₄-alkanoic acids, C₁-C₁₈-alkylmonoesters of phthalic acid, hexahydrophthalic acid, maleic acid or terephthalic acid, maleic anhydride, phthalic anhydride, hexahydrophthalic anhydride, benzenesulfonic acid, p-toluenesulfonic acid, p-dodecylbenzenesulfonic acid, alkali metal dodecyl sulfates, n-dodecane, n-alkanols having from 9 to 18 carbon atoms or salts of the acids specified.

4. A process for preparing alkoxylation products as claimed in any of claims 1 to 3, wherein the additives (a) to (j) are used in a molar ratio to the mixed hydroxides I or II of from 0.01:1 to 10:1.

5. A process for preparing alkoxylation products as claimed in any of claims 1 to 4 by reaction of C₁-C₃₀-alkanols or C₃-C₃₀-alkenols as compounds having active hydrogen atoms.

6. A process for preparing alkoxylation products as claimed in any of claims 1 to 5, wherein the mixed hydroxides I or II are used in amounts of from 0.1 to 5 % by weight, based on the weight of the compounds having active hydrogen atoms.

7. A process for preparing alkoxylation products as claimed in any of claims 1 to 6, wherein the reaction is carried out at from 80 to 230°C and pressures of from 2 to 10 bar.

8. Use of mixed hydroxides I and II built up of polycations and modified with additives as set forth in claim 1 as alkoxylation catalysts in the reaction of compounds having active hydrogen atoms with C₂-C₄-alkylene oxides.

9. A mixed hydroxide built up of polycations and having the general formula Ia or IIa
[M(II)₁₋ₓM(III)ₓ(OH)₂]A_{x/n} · m L (Ia)
[LiAl₂(OH)₆]A_{1/n} · m L (IIa)
where
M(II) is at least one divalent metal ion,
M(III) is at least one trivalent metal ion,
A is at least one inorganic anion and
L is an organic solvent or water,
n is the valence of the inorganic anion A or in the case of a plurality of anions A is their mean valence and
x can assume a value of from 0.1 to 0.5 and
m can assume a value of from 0 to 10,
which has been modified with
(a) aromatic or heteroaromatic mono- or polycarboxylic acids or their salts,
(b) aliphatic monocarboxylic acids or their salts from the group consisting of phenyl-C₂-C₄-alkanoic acids,
cyclopentanecarboxylic acid, cyclohexanecarboxylic acid, cyclohexyl-C₂-C₄-alkanoic acids, cinnamic acid, o- or p-chlorocinnamic acid and pyridylacetic acids,
(c) monoesters of dicarboxylic acids or their salts,
(d) maleic anhydride, phthalic anhydride or hexahydrophthalic anhydride,
(e) aliphatic sulfonic acids or their salts,
(f) C₈-C₁₈-alkyl sulfates,
(g) C₈-C₅₀-n-alkanes or
(j) n-alkanols having from 9 to 18 carbon atoms or phenols, which are not built in between the layers of the mixed hydroxide Ia or IIa,
as additives,
with the exception of benzoic acid and its salts in the case of a lithium-aluminum mixed hydroxide having a layer structure,
with the exception of terephthalic acid, p- and o-chlorocinnamic acid and the corresponding salts and of n-dodecyl sulfate in the case of magnesium-aluminum mixed hydroxides having a layer structure,
and also with the exception of phthalic acid, isophthalic acid, terephthalic acid and pyridinedicarboxylic acids in the case of calcium-aluminum mixed hydroxides.

10. A process for preparing mixed hydroxides Ia and IIa as claimed in claim 9, which comprises precipitating the compounds Ia or IIa under alkaline conditions from aqueous solutions of M(II) and M(III) salts or aqueous solutions of lithium and aluminum salts in the presence of the additives (a) to (g) and (j).

11. A process for preparing mixed hydroxides Ia and IIa as claimed in claim 9, which comprises reacting the unmodified mixed hydroxides with the additives (a) to (g) and (j) in the presence or absence of a solvent at from 0 to 200°C.

12. A process for preparing mixed hydroxides Ia and IIa as claimed in claim 9, which comprises heating the unmodified mixed hydroxides to > 200°C, with the temperature selected being able to be no higher than that which allows the mixed hydroxide to subsequently return completely to the original crystal structure, reacting the mixed hydroxide thus treated with the additives (a) to (g) and (j) and subsequently, if desired, further treating it with water.

## Revendications

1. Procédé de préparation de produits d'alcoxylation par réaction de composés à atomes d'hydrogène actifs avec des oxydes d'alkylène en C₂-C₄, en présence d'un hydroxyde mixte, construit à partir de polycations et modifié par des additifs, de formule générale I ou Il
[M(II)₁₋ₓM(III)ₓ(OH)₂]A_{x/n} · m L (I)
[LiAl₂(OH)₆]A_{1/n} · m L (II)
dans lesquelles
M(II) représente au moins un ion métallique bivalent,
M(III) représente au moins un ion métallique trivalent,
A représente au moins un anion inorganique et
L représente un solvant organique ou de l'eau,
n représente la valence de l'anion inorganique A ou lorsqu'il y a plusieurs anions A, leur valence moyenne et
x peut prendre une valeur allant de 0,1 à 0,5 et
m peut prendre une valeur allant de 0 à 10,
en tant que catalyseur d'alcoxylation, caractérisé en ce que l'hydroxyde mixte contient comme additifs
(a) des acides mono- ou polycarboxyliques aromatiques ou hétéroaromatiques ou leurs sels,
(b) des acides mono- ou polycarboxyliques aliphatiques ou leurs sels avec un cycle hétérocyclique ou isocyclique dans la chaîne latérale,
(c) des hémiesters d'acides dicarboxyliques ou leurs sels,
(d) des anhydrides d'acides carboxyliques,
(e) des acides sulfoniques aliphatiques ou aromatiques ou leurs sels,
(f) des sulfates d'alkyle en C₈-C₁₈,
(g) des paraffines à longue chaîne,
(h) des polyétherols ou des polyétherpolyols ou
(j) des alcools ou des phénols, qui ne sont pas disposés entre les couches de l'hydroxyde mixte I ou Il.

2. Procédé de préparation de produits d'alcoxylation selon la revendication 1, en présence d'un hydroxyde mixte de formule générale I, dans laquelle M(II) représente le magnésium, M(III) l'aluminium et A des carbonates.

3. Procédé de préparation de produits d'alcoxylation selon la revendication 1 ou 2, caractérisé en ce que l'hydroxyde mixte I ou II contient en tant qu'additifs des acides benzénemonocarboxyliques ou benzènedicarboxyliques, pour lesquels le noyau benzénique peut en outre être substitué par un à trois groupements alkyle en C₁-C₄, des acides phényl(alcanoïque en C₂-C₄), l'acide cyclohexanecarboxylique, des acides cyclohexyl(alcanoïque en C₂-C₄), des hémiesters d'alkyle en C₁-C₁₈ d'acide phtalique, d'acide hexahydrophtalique, d'acide maléique ou d'acide téréphtalique, de l'anhydride maléique, de l'anhydride phtalique, de l'anhydride hexahydrophtalique, de l'acide benzènesulfonique, de l'acide p-toluènesulfonique, de l'acide p-dodécylbenzènesulfonique, des sulfates de dodécyle et de métaux alcalins, du n-dodécane, des n-alcanols ayant 9 à 18 atomes de carbone ou des sels des acides cités.

4. Procédé de préparation de produits d'alcoxylation selon l'une quelconque des revendications 1 à 3, caractérisé en ce que l'on introduit les additifs (a) à (j) dans un rapport molaire aux hydroxydes mixtes I ou II de 0,01:1 à 10:1.

5. Procédé de préparation de produits d'alcoxylation selon l'une quelconque des revendications 1 à 4, par réaction d'alcanols en C₁-C₃₀ ou d'alcénols en C₃-C₃₀ en tant que composés à atomes d'hydrogène actifs.

6. Procédé de préparation de produits d'alcoxylation selon l'une quelconque des revendications 1 à 5, caractérisé en ce que l'on introduit les hydroxydes mixtes I ou II en une quantité de 0,1 à 5% en poids, par rapport au poids des composés à atomes d'hydrogène actifs.

7. Procédé de préparation de produits d'alcoxylation selon l'une quelconque des revendications 1 à 6, caractérisé en ce que l'on mène la réaction à des températures de 80 à 230°C et sous des pressions de 2 à 10 bar.

8. Utilisations d'hydroxydes mixtes I ou II construits à partir de polycations et modifiés par des additifs selon la revendication 1 en tant que catalyseurs d'alcoxylation lors de la réaction de composés à atomes d'hydrogène actifs avec des oxydes d'alkylène en C₂-C₄.

9. Hydroxydes mixtes, construits à partir de polycations, de formule générale la et IIa
[M(II)₁₋ₓM(III)ₓ(OH)₂]A_{x/n} · m L (Ia)
[LiAl₂(OH)₆]A_{1/n} · m L (IIa)
dans lesquelles
M(II) représente au moins un ion métallique bivalent,
M(III) représente au moins un ion métallique trivalent,
A représente au moins un anion inorganique et
L représente un solvant organique ou de l'eau,
n représente la valence de l'anion inorganique A ou lorsqu'il y a plusieurs anions A, leur valence moyenne et
x peut prendre une valeur allant de 0,1 à 0,5 et
m peut prendre une valeur allant de 0 à 10,
qui sont modifiés par
(a) des acides mono- ou polycarboxyliques aromatiques ou hétéroaromatiques ou leurs sels,
(b) des acides monocarboxyliques aliphatiques ou leurs sels choisis dans le groupe formé par les acides phényl(alcanoïque en C₂-C₄), l'acide cyclopentanecarboxylique, l'acide cyclohexanecarboxylique, les acides cyclohexyl(alcanoïque en C₂-C₄), l'acide cinnamique, l'acide o- ou p-chlorocinnamique et les acides pyridylacétiques,
(c) des hémiesters d'acides dicarboxyliques ou leurs sels,
(d) l'anhydride maléique, l'anhydride phtalique ou l'anhydride hexahydrophtalique,
(e) des acides sulfoniques aliphatiques ou leurs sels,
(f) des sulfates d'alkyle en C₈-C₁₈,
(g) des n-alcanes en C₈-C₅₀, ou
(j) des n-alcanols ayant 9 à 18 atomes de carbone ou des phénols, qui ne sont pas disposés entre les couches de l'hydroxyde mixte la ou IIa
en tant qu'additifs,
à l'exception de l'acide benzoïque et de ses sels dans le cas d'un hydroxyde mixte lithium/aluminium à structure stratifiée,
à l'exception de l'acide téréphtalique, des acides p- et o-chlorocinnamique et des sels correspondants et du sulfate de n-dodécyle dans le cas d'hydroxydes mixtes magnésium/aluminium à structure stratifiée,
ainsi qu'à l'exception des acides phtalique, isophtalique, téréphtalique et pyridinecarboxyliques dans le cas d'hydroxydes mixtes calcium/aluminium.

10. Procédé de préparation d'hydroxydes mixtes la et IIa selon la revendication 9, caractérisé en ce que l'on fait précipiter de façon alcaline les composés la ou IIa à partir de solutions aqueuses de sels de M(II) et M(III) respectivement de solutions aqueuses de sels de lithium et d'aluminium en présence des additifs (a) à (g) et (j).

11. Procéde de préparation d'hydroxydes mixtes la et IIa selon la revendication 9, caractérisé en ce que l'on fait réagir les hydroxydes mixtes non modifiés avec les additifs (a) à (g) et (j) en présence ou en l'absence d'un solvant à des températures de 0 à 200°C.

12. Procédé de préparation d'hydroxydes mixtes la et IIa selon la revendication 9, caractérisé en ce que l'on chauffe les hydroxydes mixtes non modifiés à des températures > 200°C, la température devant être choisie juste assez haute pour que l'hydroxyde mixte puisse retrouver ensuite entièrement sa structure cristalline de départ, on fait réagir l'hydroxyde mixte ainsi traité avec les additifs (a) à (g) et (j) et l'on traite ensuite éventuellement avec de l'eau.
